# EUROPEAN PATENT APPLICATION

(11) **EP 2 153 822 A1**
(43) Date of publication of application: **17.02.2010**
(21) Application number: 08162297.9
(22) Date of filing: 13.08.2008
(51) Int. Cl.: A61K 9/16, A61K 9/20, A61K 31/4178, A61K 31/54

(54) **Granulation of active pharmaceutical ingredients**

(71) Applicant: Lek Pharmaceuticals D.D., 1526 Ljubljana (SI)
(72) Inventor: The designation of the inventor has not yet been filed
(74) Representative: Kosak, Alenka

(57) **Abstract**

The present invention discloses a dry formulation or granulation comprising, in admixture, more than 50 wt.% of active pharmaceutical ingredient and from 1 to 10 wt.% of nonaqueous excipient selected from liquid substances. In a preferred embodiment no water or lower alcohol has been added in any step of the preparation of the dry formulation or granulation, and the nonaqueous excipient selected from liquid substances is adsorbed to dryness by the active pharmaceutical ingredient and/or by a further solid excipient.

Pharmaceutical formulations based on such dry formulation or granulation, and processes for the preparation thereof, are described as well. The dry formulations or granulations reliably and effectively further processed under dry conditions without requiring active drying, including e.g. briquetting, slugging, sieving, milling, tabletting, further fine granulating, direct compression, and the like.

## Description

### Field of the invention

The present invention relates to a new granulation technique, which is particularly suitable for the preparation of high dosage formulations of active pharmaceutical ingredients (API). The present invention also relates to new formulations or granulations as well as pharmaceutical formulations resulting therefrom.

### Description of the background art

Granulation techniques are commonly applied processes to formulate pharmaceutical preparations. These techniques are often used to formulate single dosage forms and pharmaceutical compositions suitable for oral administration. Such techniques are generally classified in wet granulation and dry granulation. Further processing steps of primarily granulated or briquetted materials may include subsequent granulation, tabletting and/or direct compression steps.
Wet granulation using substantial amounts of wetting agent is the method which is most commonly used in the pharmaceutical industry, as it provides better prospects in terms of ease of processing, especially with respect to the required flow and cohesive properties, and presumably less problems associated with physical characteristics of various ingredients in the formulation. Dry granulation and optionally performed direct compression on the other hand do not need an agglomeration liquid, such as water or aqueous lower alcohol (normally ethanol-based) solution, but problems such as poor flowability and inadequate compressibility have to be encountered, especially if a high API load or API dosage in the dry formulation or the final pharmaceutical composition is desired while the selected API does not have suitable characteristics as a powder.

It was thus an object to provide an improved process for the preparation of a pharmaceutical formulation, as well as to provide an improved pharmaceutical formulation suitable for high API load.

### Summary of the invention

According to an aspect of the present invention, there is provided a dry formulation or granulation comprising, in admixture, more than 50 wt.% of active pharmaceutical ingredient and from 1 to 10 wt.% of nonaqueous excipient selected from liquid substances.

According to further aspects of the present invention, there is provided a dry formulation or granulation comprising, in admixture and without water or lower alcohol having been added in any step of its preparation, (i) more than 50 wt.% of active pharmaceutical ingredient and (ii) a nonaqueous excipient selected from liquid substances; wherein the liquid nonaqueous excipient is adsorbed to dryness by the active pharmaceutical ingredient and/or by a further solid excipient.

According to further aspects of the present invention, there is provided a process for the preparation of a pharmaceutical formulation, comprising mixing more than 50 wt.% of active pharmaceutical ingredient with from 1 to 10 wt.% of a nonaqueous liquid excipient to obtain a dry or moisture-activated formulation, and subjecting the thus obtained formulation to further processing to prepare the pharmaceutical formulation.

According to further aspects of the present invention, there is provided another process for the preparation of a pharmaceutical formulation, comprising mixing active pharmaceutical ingredient with a nonaqueous liquid excipient, without adding anyone of water, aqueous solution, lower alcohol or lower alcoholic solution, as granulation liquid, wherein the nonaqueous liquid excipient and other optional solid excipients each has a water activity of less than 0.62 determined at room temperature.

In another aspect, the present invention provides a pharmaceutical formulation comprising a dry formulation or granulation, or obtained from the process embodiments according to any of the above aspects.

The present invention also provides the dry formulation or granulation, or the pharmaceutical formulation according to any of the above aspects for use in the treatment of human or animal disease.

### Brief description of the drawings

- Fig. 1: shows the effect of various non-aqueous liquid excipients and their contents on the flowability of powder mixtures for bricketing;
- Fig. 2: shows the effects of various non-aqueous liquid excipients and their contents on the compressibility of powder mixtures for tabletting.
- Figs. 3A and 3 B: show dissolution profiles of an exemplified API from pharmaceutical formulations obtained in Examples 3 and 4 at low pH (0.1 M HCl, Fig. 3A) and at approximately neutral pH (pH 7.5, Fig. 3B);

### Description of the invention, its advantages and preferred embodiments

The aspects, advantages, features and preferred embodiments of the present invention summarized in the following independent and preferred items, respectively alone or in combination, further contribute to solving the object of the invention:
(1) A dry formulation or granulation comprising, in admixture, more than 50 wt.% , preferably more than 60 wt.% of active pharmaceutical ingredient and from 1 to 10 wt.%, preferably from 2 to 9 wt.% of nonaqueous excipient selected from liquid substances.
(2) A dry formulation or granulation comprising, in admixture and without water or lower alcohol having been added in any step of preparation, more than 50 wt.%, preferably more than 60 wt.% of active pharmaceutical ingredient and a nonaqueous excipient selected from liquid substances; wherein the liquid nonaqueous excipient is adsorbed to dryness of the formulation or granulation by the active pharmaceutical ingredient and/or by a solid excipient.
(3) The dry formulation or granulation according to item (1), wherein the nonaqueous excipient is selected from the group consisting of liquid surfactants, preferably of non-ionic type; liquid polyethylene glycol; liquid paraffin; propylene glycol; liquid fatty alcohols with at least 8 carbon atoms; liquid triglycerides or oils; liquid wax; liquid polyethoxylated fatty acids; liquid PEG glycerol fatty acid esters; and liquid ethers of polyethylene glycol and alkyl alcohols; preferably selected from the group consisting of polysorbate, glycerol, liquid polyethylene glycol, liquid paraffin and propylene glycol.
(4) The dry formulation or granulation according to any one of items (1) to (3), further comprising a solid excipient capable of adsorbing the orginally liquid nonaqueous excipient.
(5) The dry formulation or granulation according to any one of items (1) to (4), further comprising a solid excipient selected from microcrystalline cellulose, lactose, isomalt, solid polyethylene glycol, mannitol, sorbitol, starch, calcium phosphates, carboxymethylcellulose (calcium or sodium), cellulose, silicified microcrystalline cellulose, cellulose acetate, colloidal silicon dioxide, dextranes, dextrin, glucose, ethylcellulose, fructose, hydroxyethyl cellulose, hydroxyethylmethyl cellulose, hydroxypropyl cellulose, hypromellose, lactitol, magnesium carbonate, maltodextrin, maltose, methylcellulose, polydextrose, pregelatinized starch, zein, and calcium silicate, preferably selected from solid polyethylene glycol, isomalt, microcristalline cellulose, mannitol and lactose.
(6) The dry formulation or granulation according to item (2), wherein the originally liquid, nonaqueous excipient is contained in the whole formulation at an amount of 1 to 10 wt.%.
(7) The dry formulation or granulation according to item (5), wherein the selected solid polyethylene glycol has molecular weight in the range of 4000 to 20000, preferably is PEG-4000.
(8) The dry formulation or granulation according to any one of the preceding items, further comprising solid excipient selected from mannitol, lactose and microcrystalline cellulose.
(9) The dry formulation or granulation according to any one of the preceding items, being obtained from using formulation or granulation liquid free of water.
(10) The dry formulation or granulation according to any one of the preceding items, wherein no water, aqueous solution, lower alcohol or lower alcoholic solution has been added to the dry formulation during its preparation.
(11) The dry formulation or granulation according to any one of the preceding items, comprising 2 to 9 wt.%, preferably 3 to 8 wt.% of the originally liquid, nonaqueous excipient.
(12) The dry formulation or granulation according to any one of the preceding items, wherein the originally liquid, nonaqueous excipient is mixed with solid excipient at a weight ratio of 1:10 to 10:1, preferably 1:2 to 8:1.
(13) The dry formulation or granulation according to any one of the preceding items, wherein polysorbate is admixed with solid polyethylene glycol at a weight ratio of 1:2 to 5:1; or wherein liquid polyethylene glycol is admixed with solid polyethylene glycol at a weight ratio of 1:1 to 8:1; or wherein propylene glycol is admixed with solid polyethylene glycol at a weight ratio of 1:2 to 4:1; or wherein glycerol is admixed with solid polyethylene glycol weight at a ratio of 1:1 to 6:1; or wherein liquid paraffin is admixed with solid polyethylene glycol weight at a ratio of 1:2 to 4:1.
(14) The dry formulation or granulation according to any one of the preceding items, wherein the originally liquid, nonaqueous excipient is liquid polyethylene glycol selected from the group consisting of PEG-200, PEG-400 and PEG-600, preferably PEG-400.
(15) The dry formulation or granulation according to any one of the preceding items, wherein the originally liquid, nonaqueous excipient is polysorbate selected from the group consisting of polysorbate-20, polysorbate-60, polysorbate-65, polysorbate-80 and polysorbate-85, preferably polysorbate-80.
(16) The dry formulation or granulation according to any one of the preceding items, wherein the originally liquid, nonaqueous excipient and the amount thereof is selected from anyone of the group consisting of 4.5 to 7 wt.% polysorbate 80; 4 to 6.5 wt.% PEG-400; 4 to 6.5 wt.% propylene glycol; 6 to 8 wt.% glycerol; and 3 to 7 wt.% liquid paraffin.
(17) The dry formulation or granulation according to any one of the preceding items, wherein active pharmaceutical ingredient is selected according to any one of the following conditions, either alone or in combination: (i) flowability below 0.15g/sec; (ii) solubility at pH of 2 below 0.1mg/ml; (iii) compressibility more than 30%; (iv) capability of existing in at least two polymorphic forms; and (v) being hygroscopic.
(18) The dry formulation or granulation according to any one of the preceding items, wherein active pharmaceutical ingredient is selected from the group consisting of eprosartan, irbesartan, amoxiciline, levofloxacine, metformin and sevelamer, their salts, hydrates or solvates, respectively optionally combined with another active pharmaceutical ingredient.
(19) The dry formulation or granulation according to item (18), wherein active pharmaceutical ingredient is eprosartan mesylate, optionally combined with hydrochlorothiazide.
(20) The dry formulation or granulation according to item (18), wherein active pharmaceutical ingredient is metformin, optionally combined with rosiglitazone.
(21) The dry formulation or granulation according to any one of the preceding items, comprising more than 60%, more preferably more than 70 % eprosartan mesylate.
(22) A process for the preparation of a pharmaceutical formulation, comprising mixing more than 50 wt.% of active pharmaceutical ingredient with from 1 to 10 wt.% of a nonaqueous liquid excipient to obtain a dry or moisture-activated formulation, and subjecting the thus obtained formulation to processing to prepare the pharmaceutical formulation.
(23) A process for the preparation of a pharmaceutical formulation, comprising mixing more than 50 wt.%, preferably more than 60 wt.% of active pharmaceutical ingredient with nonaqueous liquid excipient, together with a solid excipient capable of adsorbing the originally liquid nonaqueous excipient and optionally other solid pharmaceutically acceptable excipients, to thereby obtain dry pharmaceutical formulation without a drying step.
(24) A process for the preparation of a pharmaceutical formulation, comprising subjecting an active pharmaceutical ingredient to granulation with a nonaqueous liquid excipient, without adding water or an aqueous solution and optionally also without low alcohol or its solution as granulation liquid, wherein the nonaqueous liquid excipient and optional other excipients each has a water activity of less than 0.62, preferably less than 0.60 and more preferably less than 0.50, respectively determined at room temperature.
(25) The process according to any one of items (22) to (24), wherein the nonaqueous excipient is selected from the group consisting of liquid surfactants, preferably of non-ionic type; liquid polyethylene glycol; liquid paraffin; propylene glycol; liquid fatty alcohols with at least 8 carbon atoms; liquid triglycerides or oils; liquid wax; liquid polyethoxylated fatty acids; liquid PEG glycerol fatty acid esters; and liquid ethers of polyethylene glycol and alkyl alcohols; preferably selected from the group consisting of polysorbate, glycerol, liquid polyethylene glycol, liquid paraffin and propylene glycol, alone or together with other pharmaceutically acceptable excipients.
(26) The process according to any one of items (22) to (25), wherein said nonaqueous liquid excipient is polysorbate and/or liquid polyethylene glycol.
(27) The process according to any one of items (22), to (26), wherein active pharmaceutical ingredient and a nonaqueous liquid excipient are granulated with a solid excipient selected from the group consisting of microcrystalline cellulose, lactose, isomalt, solid polyethylene glycol, mannitol, sorbitol, starch, calcium phosphates, carboxymethylcellulose (calcium or sodium), cellulose, silicified microcrystalline cellulose, cellulose acetate, colloidal silicon dioxide, dextranes, dextrin, glucose, ethylcellulose, fructose, hydroxyethyl cellulose, hydroxyethylmethyl cellulose, hydroxypropyl cellulose, hypromellose, lactitol, magnesium carbonate, maltodextrin, maltose, methylcellulose, polydextrose, pregelatinized starch, zein, and calcium silicate, preferably a solid excipient selected from solid polyethylene glycol, isomalt, microcristalline cellulose, mannitol and lactose.
(28) The process according to any one of items (22) to (27), wherein 2 to 9 wt.% of nonaqueous liquid excipient is used.
(29) The process according to any one of items (22) to (28), wherein the nonaqueous liquid excipient is liquid polyethylene glycol selected from the group consisting of PEG-200, PEG-400 and PEG-600, and/or is polysorbate selected from the group consisting of polysorbate-20, polysorbate-60, polysorbate-65, polysorbate-80 and polysorbate-85.
(30) The process according to item (29), wherein the liquid polyethylene glycol is PEG-400 and/or wherein the polysorbate is polysorbate-80.
(31) The process according to any one of items (22) to 30), wherein solid polyethylene glycol is added.
(32) The process according to any one of items (22) to (31), wherein a weight ratio of nonaqueous liquid excipient and solid excipient is adjusted in a range of 1:1 to 10:1.
(33) The process according to any one of items (22) to (32), further comprising briquetting or slugging, then optionally sieving or milling, and subsequently tabletting, to thereby obtain single dosage forms, preferably tablets.
(34) The process according to any one of items (22) to (33), and subjecting said primary granulation to a direct compression or a dry granulation process.
(35) The process according to any one of items (22) to (34), wherein, while the active pharmaceutical ingredient has a property of existing in at least two polymorphic forms, the actual active pharmaceutical ingredient subjected to granulation is provided in a single first form, wherein the mixing or granulation step is performed such that an interconversion from said first form to another or mixed form of the active pharmaceutical ingredient is prevented.
(36) The process according to item (35), wherein the at least two polymorphic forms of the active pharmaceutical ingredient are selected from hydrate forms, anhydrate form and solvate forms, wherein the provided single first form preferably is the anhydrous form.
(37) The process according to item (35), wherein optional further steps of briquetting or slugging, sieving or milling, tabletting or direct compression are further processed without water and avoiding aqueous moisture to thereby maintain said single first form of the active pharmaceutical ingredient.
(38) The process according to any one of items (35) to (37), wherein said first form of the active pharmaceutical ingredient which is subjected to granulation is anhydrous form, which is prevented from interconversion into hydrate forms.
(39) The process according to any one of items (22) to (38), wherein active pharmaceutical ingredient is selected according to any one of the following conditions, either alone or in combination: (i) flowability below 0.15g/sec; (ii) solubility at pH of 2 below 0.1mg/ml; (iii) compressibility more than 30%; (iv) capability of existing in at least two polymorphic forms; and (v) being hygroscopic.
(40) The process according to any one of items (22) to (39), wherein active pharmaceutical ingredient is selected from a group of eprosartan, irbesartan, amoxiciline, levofloxacine, metformin and sevelamer, their salts, hydrates or solvates, optionally combined with another active pharmaceutical ingredient.
(41) The process according to item (40), wherein active pharmaceutical ingredient is eprosartan mesylate, optionally combined with hydrochlorothiazide.
(42) The process according to item (40), wherein active pharmaceutical ingredient is metformin, optionally combined with rosiglitazone.
(43) The process for the preparation of a dry pharmaceutical formulation according to any one of the items (22) to (42), further comprising tabletting.
(44) A pharmaceutical formulation, comprising, in admixture, more than 50 wt. %, preferably more than 60 wt.%, particularly more than 70 wt. % of active pharmaceutical ingredient and 1 to 10 wt.%, preferably 2 to 9 wt.% of nonaqueous excipient selected from liquid substances.
(45) The pharmaceutical formulation according to item (44), wherein the liquid nonaqueous excipient is selected from the group consisting of liquid surfactants, preferably of non-ionic type; liquid polyethylene glycol; liquid paraffin; propylene glycol; liquid fatty alcohols with at least 8 carbon atoms; liquid triglycerides or oils; liquid wax; liquid polyethoxylated fatty acids; liquid PEG glycerol fatty acid esters; and liquid ethers of polyethylene glycol and alkyl alcohols; preferably selected from the group consisting of polysorbate, glycerol, liquid polyethylene glycol, liquid paraffin and propylene glycol.
(46) The pharmaceutical formulation, comprising, in admixture and without water or lower alcohol having been added in any step of preparation, more than 50 wt. %, preferably more than 60 wt.%, particularly more than 70 wt. % of active pharmaceutical ingredient and a nonaqueous liquid excipient, which as such was liquid when originally added, wherein the liquid nonaqueous excipient is adsorbed to dryness of the formulation by the active pharmaceutical ingredient and/or by a solid excipient.
(47) The pharmaceutical formulation according to any one of items (44) to (46), comprising a solid excipient selected from the group consisting of microcrystalline cellulose, lactose, isomalt, solid polyethylene glycol, mannitol, sorbitol, starch, calcium phosphates, carboxymethylcellulose (calcium or sodium), cellulose, silicified microcrystalline cellulose, cellulose acetate, colloidal silicon dioxide, dextranes, dextrin, glucose, ethylcellulose, fructose, hydroxyethyl cellulose, hydroxyethylmethyl cellulose, hydroxypropyl cellulose, hypromellose, lactitol, magnesium carbonate, maltodextrin, maltose, methylcellulose, polydextrose, pregelatinized starch, zein, and calcium silicate; preferably selected from solid polyethylene glycol and isomalt.
(48) The pharmaceutical formulation according to any one of items (44) to (47), obtained by direct compression or dry granulation.
(49) The pharmaceutical formulation according to any one of items (44) to (48), wherein active pharmaceutical ingredient is selected according to any one of the following conditions, either alone or in combination: (i) flowability below 0.15g/sec; (ii) solubility at pH of 2 below 0.1mg/ml; (iii) compressibility more than 30%; (iv) capability of existing in at least two polymorphic forms; and (v) being hygroscopic - for example determined by absorption of significant amounts (at least 10wt-%, or even at least 25%) of atmospheric moisture when exposed to conditions of relative humidity (RH) of at least 60 % RH.
(50) The dry formulation or granulation according to any one of items (44) to (49), wherein active pharmaceutical ingredient is selected from a group of eprosartan, irbesartan, amoxiciline, levofloxacine, metformin and sevelamer, their salts, hydrates or solvates, optionally combined with another active pharmaceutical ingredient.
(51) The pharmaceutical formulation according to item (50), wherein the active pharmaceutical ingredient is eprosartan mesylate, optionally combined with hydrochlorothiazide.
(52) The pharmaceutical formulation according to item (50), wherein the active pharmaceutical ingredient is metformin, optionally combined with rosiglitazone.
(53) The pharmaceutical formulation according to any one of items (44) to (52), which is a tablet.
(54) A tablet according to item (53), which has a hardness of between 40 and 180 N, preferably between 80 and 140 N, more preferably from 100 to 120 N.
(55) A package comprising: at least one single dosage form comprising a pharmaceutical formulation according to any one of items (44) to (54); wherein said at least one single dosage form is packed in a package sealed against vapor and moisture permeation, and preferably further protected against light exposure.
(56) Use of dry formulation or granulation according to any of items (1) to (21), or pharmaceutical formulation according to any of items (44) to (52), or a tablet according to item (53) or (54) for the manufacture of a medicament.
(57) Dry formulation or granulation according to any of items (1) to (21), or pharmaceutical formulation according to any of items (44) to (52), or a tablet according to item (53) or (54) for use in the treatment of human or animal disease.
(58) The formulation or granulation according to item (57), wherein the human or animal disease is determined by the conventional use of the selected active pharmaceutical ingredient.
(59) The formulation or granulation according to item (19), or pharmaceutical formulation of item (40) for use in the treatment of hypertension.
(60) The formulation or granulation according to item (20), or pharmaceutical formulation of item (41) for use in the treatment of diabetes.
(61) The process for the preparation of a dry pharmaceutical formulation according to any one of the items (22) to (43), wherein a time of mixing after the liquid nonaqueous excipient has been added to the active pharmaceutical ingredient, and before additional solid excipients are further added, is below 10 minutes, preferably 2 to 5 minutes, particularly around 3 minutes.

Surprisingly, it was found that the use of a non-aqueous excipient, which is liquid when admixed with an active pharmaceutical ingredient (API), can remarkably enhance flowability of the API mixture even if the API is at a relatively high load. Further unexpected enhancements of performances despite of high API load of more than 50wt.-%, optionally also more than 60wt.-% and even more than 70wt.-%, include significantly increased flowability, preferably to ranges of at least 0.5 g/s, substantially increased compressibility, preferably to ranges of e.g. 15-20% of the final granulate, while at the same time balancing excellent tablet characteristics such as a combination of hardness, friability, weight, shape, disintegration and dissolution properties, are achievable by the present invention.

Flowability and compressibility characteristics are particularly enhanced when the amount of the originally liquid, non-aqueous excipient that is admixed with the API is carefully controlled to a range of 1 to 10 wt.-%, preferably 2 to 9 wt.%, and more preferably 3 to 8 wt.% relative to the API-containing formulation or granulation. This especially applies to cases when according to preferred embodiments the originally liquid, non-aqueous excipient is selected from the group consisting of polysorbate, glycerol, liquid polyethylene glycol, liquid paraffin and propylene glycol.

According to the present invention, use can be made of an advantage that, if desired and to the additional benefit of API stability especially at high API load, the formulation or granulation of the admixture of originally liquid, non-aqueous excipient and API can be processed without an addition of water, an aqueous solution or an aqueous suspension, and likewise without lower alcohol additions or solutions like those of methanol and ethanol. It is within the scope of the present invention, and within certain embodiments even preferred in terms of improved flowability and compressibility at high API load, that the liquid nonaqueous excipient becomes adsorbed by other components of the admixture such as the highly loaded API and/or optional solid excipients, and thus the whole formulation and granulation may become "dry" or at least "moisture-activated dry". The present invention significantly distinguishes over a conventional modification of dry granulation, also known as and sometimes referred to as "moisture activated dry granulation" (MADG). The conventional MADG is described for example by Christensen et al. in "Drug Development and Industrial Pharmacy" 20(14), pp. 2195-2213 (1994), in Encyclopedia of Pharmaceutical Technology, Vol. 4, J. Swarbrick and J.C. Boylan (eds.) Marshall Decker Inc., New York-Basel-Hong Kong, pp. 102-104 (1991) and by S. Inghilbrecht and J.P. Remon in International Journal of Pharmaceutics 171, pp. 195-206 (1998), and is based on the addition of limited amount of water to a dry blend of drug, binder and filler without the need of a drying step being involved. In comparison to conventional MADG, the use according to the present invention of non-aqueous liquid instead of limited water in an otherwise dry mixing system, which may be denoted "non-aqueous moisture activated dry granulation" (NAMADG), has been specifically developed for cases of high API load.

The NAMADG principle according to the present invention can be used as a standalone process for the preparation of a primary formulation or granulation, and it can be used also in combination with further downstream processing techniques. In particular, it has been found that the primary granulation undergoing NAMADG provides API at high load with good cohesive properties, well suited for further dry processing such dry granulation in general and briquetting, sieving, milling in particular, and/or for further direct compression. Further advantages of the NAMADG concept of the present invention over prior art wet or dry granulation techniques include uniformity of blend, involvement of less manufacturing steps and hence less cost, elimination of heat and moisture, prime particle dissociation, and physical stability.

The expression "dry formulation or granulation" used herein means that the non-aqueous, originally liquid excipient is used and processed together with API present as the main component, and that the amount of the non-aqueous, originally liquid excipient is limited so as to avoid solution or suspension states of the whole formulation or granulation. Although mixing of the ingredients may proceed through wet massing within a suitable time for non-aqueous moisture-activation, typically dry mixing follows after absorption of the limited amount of liquid excipient, whereupon the formulation or granulation obtained can be further processed under dry conditions without a need for active drying, including dry mass processing of e.g. briquetting, slugging, sieving, milling, tabletting, further fine granulating, direct compression, and the like. Respective amounts of non-aqueous, originally liquid excipient, of other solid excipients and of API disclosed herein are referred to the whole amount of the formulation or granulation in which these components are co-processed. The amount indication also applies to products obtained by further co-processing of the aforementioned components, including but not limited to briquetting, slugging, sieving, milling, tabletting, further fine granulating and direct compression, and correspondingly applies to a final pharmaceutical formulation. In this co-processing, the API can be a single compound or multiple different compounds, and also the non-aqueous, originally liquid excipient and other solid excipients can be a single compound or a mixture, respectively. When according to further embodiments additional excipients or API, either the same or different as used in the common formulation or granulation, are further included into separate compartments or parts of a dosage form, such as extragranulary or in separate tablet layers (e.g. present in bilayer or trilayer tablets), in one or more coatings on cores, or the like, the respective amounts of such additional material are disregarded in the presently disclosed amounts of the originally liquid non-aqueous excipient and the API which are present, in admixture, in the common formulation or granulation. For example, the amount indications would apply to a tablet core, which optionally may itself be composed of intragranular and external constituents, but not to optional coatings thereon. This is because the admixture unit comprising the relevant excipients and API disclosed herein is relevant for the flowability and compressibility as well as additional advantageous effects of the present invention.

The admixing of API and nonaqueous excipient disclosed herein is preferably performed with a high shear mixer. The use of liquid, non-aqueous excipient has been found to be consistent with a high shear mixer for producing granulations at high API load, which are subsequently suitable for tabletting and/or for direct compression.

The term "liquid, non-aqueous excipient" used herein means a material that is liquid as such, i.e. being liquid without further solvent addition such as water, aqueous solutions, low alcohol solutions such as methanol or ethanol solutions or the like, and is generally understood to include fluids, flowing materials, low viscous, medium viscous up to high viscous materials. It is sufficient that the non-aqueous excipient is, in this general meaning, "liquid" in the step of mixing with the API compound and at the applied operation temperature. But the liquid nature should preferably exist at room temperature, because this allows the process according to the present invention to proceed without active heating. During or after the mixing with the API, the originally liquid non-aqueous excipient may be adsorbed, if not already by the highly loaded API component, by other excipients such as binders, fillers or other dry excipient materials described later, to thereby eventually form a dry formulation or granulation that is optionally further processed. Further processing may include, without being limited to, bricketing or slugging, optionally sieving or milling, and tableting or direct compression, to typically obtain single dosage forms, preferably tablets.

Suitable examples of originally liquid, non-aqueous excipients include, but are not limited to liquid surfactants, preferably of non-ionic type, such as liquid polyoxyethylene-polyoxypropylene block copolymers or poloxamers or pluronics, polysorbate, liquid sorbitan fatty acid esters (Spans^{®}); liquid polyoxyethylene fatty acid glyderides (Cremophors^{®} such as liquid Cremophor EL^{®}), vitamin E TPGS (TPGS 1000), polyoxyethylene fatty acid esters (Myrjs^{®}); liquid polyethylene glycol; liquid paraffin; propylene glycol; liquid fatty alcohols with at least 8, preferably at least 12 carbon atoms; liquid triglycerides or oils such as petroleum, soybean oil, mineral oil, peanut oil, castor oil, coconut oil, corn oil, cottonseed oil, olive oil, palm oil, rapeseed oil, sesame oil, sunflower oil; liquid wax; liquid polyethoxylated fatty acids such as PEG-laurate, PEG-oleate and PEG-stearate; liquid PEG glycerol fatty acid esters such as PEG-glyceryl laurate and PEG-glyceryl oleate; ethers of polyethylene glycol and alkyl alcohols such as PEG-oleyl ether and PEG-lauryl ether; and mixtures thereof. Preferred liquid nonaqueous excipients are selected from the group consisting of polysorbate, glycerol, liquid polyethylene glycol, liquid paraffin and propylene glycol. Polysorbate is preferably selected from the group consisting of polysorbate-20, polysorbate-60, polysorbate-65, polysorbate-80 and polysorbate-85, more preferably polysorbate-80. Liquid polyethylene glycol preferably is selected from the group consisting of PEG-200, PEG-400 and PEG-600, more preferably PEG-400.

In combination with a solid excipient capable of effectively adsorbing the nonaqueous liquid excipient, especially when selecting the solid excipient from solid polyethylene glycol, isomalt, microcristalline cellulose (e.g. Avicel), mannitol and lactose, the non-aqueous excipient beneficially may be chosen more freely among those providing liquid forms, i.e. may include the use of liquid non-aqueous excipient other than the afore-mentioned preferred ones, and further allows a higher variability of the amount of the liquid nonaqueous excipient while still accomplishing significant contribution to effects like flowability and compressibility at high API load. Isomalt is particularly preferred as a solid excipient adsorbing the originally liquid, nonaqueous excipient. Further examples of solid excipients effectively co-acting with the non-aqueous, originally liquid excipient include solid excipients selected from mannitol, lactose and microcrystalline cellulose. Mannitol and/or lactose further enhance dissolution properties.

The preferred amount of the liquid nonaqueous excipient is a range of 1 to 10 wt.%, particularly 2 to 9 wt.% and more particularly 3 to 8 wt.%, in terms of enhancement of flowability and compressibility effects. Another preferred embodiment of this aspect is using the preferred non-aqueous, originally liquid excipients selected from the group consisting of polysorbate, glycerol, liquid polyethylene glycol, liquid paraffin and propylene glycol, more preferably in combination with a solid polyethylene glycol and/or isomalt.

Besides paying attention to selecting particular substances as described above, excipients or additives besides the API and the liquid nonaqueous excipient may be suitably selected from binder, diluent, lubricant, disintegrant, antiadhesive, glidant, alone or in combination. Further useful additives may include, alone or in combination, buffer agents, anti-oxidants, colorants, stabilizers, fillers, plasticizers, emulsifiers, preservatives, viscosity-modifying agents, or passifiers, flavouring agents, without being limited thereto.

Suitable further, preferably solid excipients include for example those selected from the group consisting of solid polyethyleneglycol (PEG; Macrogol), preferably PEGs having molecular weights in the range of 4000 to 20000; polyvinyl pyrrolidone (PVP) or its crosslinked form crospovidone; silicon dioxide, preferably fumed silica or colloidal silicon dioxide; sugar esters and polyhydroxy-sugars, preferably mannitol; lactose, isomalt, lactitol and dextrose; buffer salts, preferably anhydrous mono-, di- or tri-basic phosphate, more preferably calcium phosphate, calcium sulfate and calcium silicate; hydrophilic, anionic, cationic, zwitterionic and non-ionic surfactants and lipohilic additives such as mono-, di- or triglycerides, polyethoxylated fatty acids and fatty acid esters, preferably sodium oleate, sodium lauryl sulfate and magnesium stearate and polyglycolized glycerides; talc; polysaccharides, preferably starch and celluloses, more preferably hydroxyethylcellulose (HEC), hydroxypropylcellulose (HPC), hydroxypropyl methylcellulose (HPMC), HPMC phthalate, cyclodextrins and carbomers, in particular hydroxypropyl methylcellulose, microcrystalline cellulose (MCC), powdered cellulose; polypeptides such as gelatine; and mixtures thereof; without being limited thereto. A single dosage pharmaceutical formulation as an embodiment of the present invention may further comprise a cover film or shell, which according to a preferred embodiment is non-hygroscopic as well. In further preferred embodiments, bi- or multi-functional excipients can be used, for example, colloidal silicon dioxide (CSD) admixed with micro-crystalline cellulose (MCC), for example Prosolv^{™}.

In terms of producing tablets with excellent overall characteristics including hardness, friability, weight, shape, disintegration and dissolution, a solid excipient or additive is preferably selected from the group consisting of PEGs having a molecular weight in the range of 4000 to 20000 (in particular MACROGOL 4000); isomalt; and mannitol.

Furthermore, the use of originally liquid, non-aqueous excipient, especially in the absence of water and without addition of water or an aqueous solution or a solution of a lower alcohol (e.g. in the absence of ethanol), allows to encounter problems of API physical stability which are prone to physical changing when coming in contact with such solvents. In particular, interchanging phenomena from one to another form of API, which might exist in anhydrous and hydrated forms (mono-, di-, tri- or other multiple hydrate forms) in the final dosage form can be substantially reduced. Consequently, not only that the process according to the present invention provides a more robust, less costly and less time consuming process by omitting a drying step. Moreover, owing to a synergistic co-action when using the originally liquid, non-aqueous excipient, especially if used in controlled amounts, and when further observing a minimization or even absence of water content both in the primary dry granulation/formulation and subsequently in the final pharmaceutical formulation such as tablet, the API can be maintained in the selected form without or essentially without interconversion to another or a mixed form among anhydrate and hydrate forms during processing, to thereby improve product reproducibility. This beneficial embodiment can be preferably applied to cases where the anhydrous form is the desired and selected API form. Obtaining pharmaceutical formulations with a selected API form in a repeatedly manner significantly contributes to important properties of the final formulation, such as uniform disintegration and dissolution profiles and thus consistent and uniform bioavailability. Furthermore, disintegration and dissolution properties can be fine-tuned by the concept of the present invention in a reliable manner by selecting the appropriate starting form of the respective API being either anhydrous or mono-, di-, tri- or other hydrate forms, yet without interconversion from the selected first form to the other or mixed forms in favour of uniform product properties. These observations likewise applies when a selected API is present in solvate forms, such as being solvated as alcoholate like methanolate, ethanolate, as esterate like ethyl acetylate, or as etherate like acetonate, without being limited thereto. Which form of API is the desired or the adequate one may depend on the individual case of application, the required or desirable synthesis scheme of the API, the required or desirable isolation procedure of the API, or the required or desirable API polymorph form, all such knowledge about the required or desirable API being known to those skilled in the art.

Thus, the present invention provides in a further aspect a process for the preparation of a pharmaceutical formulation, comprising subjecting an active pharmaceutical ingredient to granulation with a nonaqueous liquid excipient, without adding water or an aqueous solution and optionally also without low alcohol or its solution as granulation liquid, wherein the nonaqueous liquid excipient and other optional excipients each has a water activity of less than 0.62 determined at room temperature.
In order to further contribute to physical stability of API and reducing the risk of the appearance of variable different anhydrous or mono- or poly-hydrated forms of API and thus variability in dissolution rates of the final solid dosage form, the liquid and/or solid excipients that are incorporated into the final solid dosage form such as tablet are carefully selected in order to have limited water activity as described above, preferably less than 0.60 and more preferably less than 0.50, respectively determined at room temperature.

By itself being independent from such limitations, it goes without saying that this aspect can be preferably combined with the other aspects of the invention, i.e. that the granulation involves the admixture of more than 50 wt.-%, preferably more than 60 wt.-% of API with a liquid non-aqueous excipient as described above, more preferable at a concentration of 1-10 wt.-% of the liquid non-aqueous excipient, particularly further involving preferred selections among liquid non-aqueous excipients and solid excipients as disclosed above.

The term "water activity" used herein is defined by the expression a_{w} = p/p₀, where p is the vapor pressure of water in the substance, and p₀ is the vapor pressure of pure water at the same temperature. Water pressure can be measured by suitable water activity meter devices, for example instrument Testo 650 using sensor 0628.0024, both available and sold by EminTech (Helsingborg, Sweden). If not otherwise stated, the water activity referred to in the present specification is determined at room temperature, which is understood herein as the temperature of 22 ± 0.5°C.

Observing the property of water activity addresses further improved characteristics, as it deals with active water molecules within the pharmaceutical formulation irrespective of its type of binding or association with different excipients or additives. The control and limitation of such active water molecules is a significant criterion for preventing unwanted interconversion of the different forms of API both during processing and particularly during storage of the final pharmaceutical formulation containing the same. Additionally, for surely maintaining physical stability over a long period of time in cases of water-sensitive and/or hygroscopic APIs, it is preferred that the water content in the tablet as a whole is minimized, suitably to less than 5%, particularly to less than 2 wt. % and more particularly to less than 0.5%.

As a further means to surely maintain physical stability over a long period of time in cases of water-sensitive and/or hygroscopic APIs, at least one of single dosage forms comprising API is packed in a package sealed against vapor and moisture permeation, and preferably further protected against light exposure. The package may be designed as a blister pack which are preferably formed with a foil/foil blister, as bottles which are preferably made mainly or completely of HDPE (high density polyethylene), or the like.

According to preferred embodiments of the present invention, performing energy consuming drying in the whole production of the dosage forms such as tablets can be beneficially omitted. Only a low number of steps may be used, such as, e.g. briquetting or slugging, optionally sieving and/or milling, and tabletting, which is in favor of process economy and costs. Simply using dry granulation or direct compression is optimally feasible. These process features together with selecting appropriate excipients and additives as described above makes the whole process of the invention more robust, economical and acceptable.

According to the present invention, the amount of filler, binder or other excipients can be limited even in cases where a direct compression or dry granulation process is used, while still providing beneficial characteristics of the final tablet. This in turn means that a high drug load can be achieved with the pharmaceutical formulations according to the present invention. This in itself is a further contribution to the achievement of an overall improved system, as in conventional wet granulation processes, excipient limitation is easier, and it is easier to provide material which has the required flow and cohesive properties to obtain an acceptable solid dosage form which do not tend to segregate.

Surprisingly, the newly developed formulation/granulation disclosed herein allows basically the same or similar sizes of tablets as that with conventional wet granulation, and at the same time the single dosage pharmaceutical formulation such as the tablets according to the invention has good physical properties and stability, yet with the possibility of yielding high weight ratios of API. Thus, the present invention meets the need of the prior art to provide an opportunity towards direct compression or dry granulation tablets containing high dose of API, thereby avoiding time and expense of wet granulation while maintaining a stable form of the active principle. As explained before, maintaining a single form of API over a long period of time ensures less variability in dissolution profiles. A further contribution of preferred embodiments according to the present invention resides in that the respective amounts of each excipient or additive may for example not exceed 10 wt.-%, preferably they may not exceed 7.5 wt.-% or even 6 wt.-%, with respect to the total formulation. Further, a majority (more than half of the excipient/additive components) of excipient or additive may not exceed 5 wt.-% each. As a consequence, the total size of the final dosage form can be properly controlled, yet containing high API load.

A further advantage of the present invention is that the various aspects and preferred embodiments disclosed herein can be applied to a variety of different API compounds. Since the concept of the present invention is particularly beneficial in such situations, the API is preferably selected with a view of critical problems associated with such selected API when formulated, that is when the API is selected according to:
(i) flowability below 0.15g/sec;
(ii) solubility at pH of 2 below 0.1mg/ml;
(iii) compressibility more than 30%;
(iv) capability of existing in at least two polymorphic forms; or
(v) being hygroscopic - for example determined by absorption of significant amounts (at least 10wt-%, or even at least 25%) of atmospheric moisture when exposed to conditions of relative humidity (RH) of at least 60 % RH;
wherein the above conditions (i) to (v) may apply either alone or in combination.

Hygroscopicity of an API or excipient can be measured by methods known to a skilled person. Mainly, such methods comprise determining the mass of small amounts of sample (i.e. 5 -10 mg) as a function of time, while varying the controlled relative humidity and/or temperature in the environment. Data on hygroscopicity of mainly used excipients can be obtained from Handbook of Pharmaceutical Excipients, 4th edition, Rowe RC, Sheskey PJ, Weller PJ, eds.; Pharmaceutical Press and American Pharmaceutical Association, London-Washington, UK-USA, 2003.

API is particularly selected in view of applications where high drug load is desired and, often associated with such high drug load, has to encounter problems with poor flowability and insufficient compression properties. Selections of particular APIs, by which favourable effects are particularly accomplished, are thus seen in the cases of using API selected from a group consisting of eprosartan, irbesartan, amoxiciline, levofloxacine, metformin and sevelamer, their salts, hydrates or solvates, optionally combined with another active pharmaceutical ingredient. As noted above, when the two or multiple APIs are co-processed in a granulation process with the originally liquid, nonaqueous excipient and optionally further excipients, their total amounts are considered to represent the high load of more than 50wt.-%, especially more than 60wt.-% and even more than 70wt.-%, whereas when only one API is co-processed but another API (or the same, but separated API) is added to another compartment or part of a pharmaceutical dosage form, only the co-processed amount of API is counted. Use of same or different APIs in different, separated parts of a solid dosage form may be suitable in cases where differential dissolution profiles are desired, e.g. including both fast and slow or delayed dissolution profiles. Because the present invention are particularly suited for such cases, the API in one preferred embodiment is eprosartan mesylate, optionally combined with hydrochlorothiazide, and in another preferred embodiment the API is metformin, optionally combined with rosiglitazone. In certain embodiments, for example when using hydrochlorothiazide or rosiglitazone in the exemplified specific embodiments, the second API is not co-processed with the first API but is added separately to the solid dosage form, for example extragranulary or in a second or third layer of a bilayer or trilayer tablet, or in coatings on a tablet core containing the first API.

Providing eprosartan mesylate at a high dose, more preferably when using stable anhydrous eprosartan mesylate, is a valuable merit of the present invention, particularly in view of the rather poor flowability characteristics of the drug *per se* being e.g. below 0.15 g/sec and thus the initially associated sticking problem on punches of tablet presses conventionally.

The following examples are merely illustrative of the present invention and they shall not be considered as limiting the scope of the invention in any way, as these examples and other equivalents thereof will become apparent to those versed in the art in the light of the present disclosure, and the accompanying claims. For illustrative purposes, the principle, advantages and preferred embodiments of the present invention are shown by the use of eprosartan mesylate as an exemplified API, while it is to be appreciated that the examples and modifications thereof are likewise applicable to other APIs.

### Example 1:

As an example, eprosatan mesylate (chemically (E)-α-[2-n-butyl-1-[(4-carboxyphenyl)methyl]-1H-imidazol-5-yl]methylene-2-thiophenepropionic acid monomethanesulfonate; an oral angiotensin II receptor (AT₁) antagonist suitable for the treatment of essential hypertension) is considered as a high dose drug for use according to an embodiment of the present invention.

Pior art processes describe the preparation of pharmaceutical formulations comprising eprosartan as follows: US 6,262,102 B1 relates to a monohydrate form, and US 2001/0031877 A1 relates to the dihydrate form of eprosartan and processes for their production, respectively. According to US 6,262,102 B1, the monohydrate form is produced during vacuum drying of the dihydrated form, or when the anhydrate is granulated with water, stored and vacuum dried. According to US 2001/0031877 A1, the dihydrated form is prepared *in situ* during the wet granulation process of the anhydrous form of the compound. The pharmaceutical formulations disclosed in the above patents are all produced by wet granulation, starting from anhydrous eprosartan mesylate, allowing the various hydrate forms to arise in situ, rendering the process unpredictable and leaving the formulation with the presence of various forms of eprosartan mesylate, namely anhydrous, monohydrate and dihydrate. In US 2002/0098241 A1, it is described that the free base form - distinct from the mesylate salt form - of anhydrous eprosartan does not form a hydrate during wet granulation, and therefore free base anhydrous form of eprosartan is proposed for a wet or (conventional) dry granulation with high drug dose. Different kind of hydrated forms in a mixture of eprosartan mesylate might have influence on *in vitro* (dissolution profiles - at infinity point) and *in vivo* profiles (bioequivalent).

Due to low solubility (i.e. less than 0.1 mg/ml at pH 2) and low permeability across the intestinal epithelium the drug exhibits very low bioavailability (13%). Therefore, an oral pharmaceutical dosage needs a high dose of eprosartan mesylate to be incorporated into the pharmaceutical formulation in order to achieve the desired biological effect (here: significant reduction of blood pressure). The recommended daily starting and usual maintainance dose of eprosartan mesylate when used as monotherapy is 735.8 mg, equivalent to 600 mg eprosartan, available as the commercial Teveten 600 mg tablets. Therefore, the amount of excipients, required to achieve satisfactory technological properties (i.e. flowability, compressibility) should be the least possible to keep the weight of the tablet as low as possible (i.e. less than 1000 mg). Solubility, flowability or compressibility can be determined by using known methods in the art, for example the methods explained in the European Pharmacopoeia, 6^{th} Edition. Larger tablets are in the light of patient compliance less preferred. WO 2006/052461 A1 proposes to enhance bioavailability of eprosartane by addition of substantial amounts of a solubilizer and an emulsifier.

Most conventional tablet formulations include a range of 60 to 74% by weight of eprosartan mesylate per tablet (Teveten^{™} and Tevetens^{™} in dosage of 300, 400 and 600 mg). This high dose drug, combined with its rather poor physical characteristics (e.g. flowability below 0.15 g/sec) has made it unsuited for direct compression as well as for dry granulation (briquetting, sieving, and tableting), and to the inventors knowledge has hitherto not allowed pharmaceutical manufactures to use direct compression as a method to prepare the final tablet.

In particular, eprosartan mesylate is hygroscopic (at over 60% RH), presents stability problems, and is not inherently compressible. Consequently, there is a need to provide a free-flowing and cohesive eprosartan mesylate composition capable of being directly compressed or dry granulated into high strengths dosage form (if possible with a desirable highest dose tablet content approximately 74% of active principal ingredient), yet with an acceptable *in vitro* dissolution profile.

The following list shows some specific data on bulk eprosartan: (particle size < 10µ) for reference:

| **Parameter** | Bulk eprosartan |
|---|---|
| Moisture (%) | 0.1 - 0.5 |
| Flow density (ml/g) | > 4.00 |
| Tapped density (ml/g) | > 3.00 |
| Compressibility (%) | > 30 |
| Flowability (g/sec) | < 0.10 |
| Angle of repose (°) | > 43 |

### Example 2:

The "nonaqueos moisture activated dry granulation" (NAMADG) principle according to an embodiment the present invention was used to produce tablets with high amount of active principle. As en exemplary API which inherently has poor to very poor flowability and insufficient compression properties, eprosatan mesylate described in Example 1 was used.

In the present basic NAMADG example using a high shear mixer for producing granulations, eprosatan mesylate at high load (73.58 wt.-%) has been processed with various liquid nonaqueous excipients in a test series at concentrations up to 10% (the weight amounts are referred to the final co-processed formulation subjected to direct compression or further dry granulation), wherein the added liquid nonaqueous excipients were selected from polysorbate (Tween^{™} 20, 60, 65, 80, 85), glycerol, liquid polyethylene glycol (macrogol; PEG-200, PEG-400, PEG-600), liquid paraffin, or propylene glycol. The mixture was blended thoroughly. Isomalt (galenlQ^{™} 720 or 721) was subsequently added to bind and sorb the small amount of nonaqueos excipient. No traditional drying step was involved. Additionally disintegrants, antiadhesives, and binders were added and blended. These samples were sieved twice (0.7 mm) and were then mixed with laboratory blender for one minute with mixture of magnesium stearate and sodium stearyl fumarate (0.3 mm). Subsequently, mixture of powders was used for compressing into briquettes using a rotary tablet press in a controlled environment. After that, briquettes were grinding twice through 2.0 mm and then 1.2 mm sieve. Afterward, external excipients were screened through a 0.3 mm screen, added to granulate, and mixed for 3 minutes. The resulting final blend was compressed into tablets using a rotary tablet press in a controlled environment. Tablets were compressed at a compression weight of 1000 mg using capsule-shaped, biconvex punches.

The whole set of ingredients of the compositions prepared in the present test series are set out in the following Table 1.

**Table 1: Composition of tablets mixture.**

| **Composition** **Internals** | **Content** **[%]** | **Function** |
|---|---|---|
| API (Eprosartan - d(0.9) = 10 µm) | 73.58 | Active |
| Nonaqueos liquid excipient* | Up to 10.00 | Binder, Wetting agent, Granulation agent |
| Macrogol 4000 (PEG-4000) | Up to 4.00 | Binder |
| Isomalt (galenIQ721) | Up to 5.00 | Filler |
| Crospovidone (Polyplasdone XL) | 3.00 - 5.00 | Disintegrant |
| Magnesium stearate | Up to 1.50 | Lubricant |
| Talc | Up to 1.50 | Glidant |
| Colloidal silicon dioxide (Aerosil 200) | Up to 1.50 | Glidant |
| Sodium stearyl fumarate (Pruv) | Up to 1.50 | Lubricant |

| Constituent **Externals** | | |
|---|---|---|
| Crospovidone (Polyplasdone XL) | 0.50 - 3.00 | Disintegrant |
| Macrogol 4000 (PEG-4000) | Up to 2.00 | Binder |
| Isomalt (galenIQ721) | Up to 2.00 | Filler |
| Colloidal silicon dioxide (Aerosil 200) | Up to 0.50 | Glidant |
| Talc | Up to 0.50 | Glidant |
| Magnesium stearate | Up to 0.50 | Lubricant |

| | | |
|---|---|---|
| ** polysorbate (Tween^{™} 20, 60, 65, 80, 85), glycerol, macrogol (PEG-200, PEG-400, PEG-600), liquid paraffin, or propylene glycol* | | |

### Examples 3 to 7:

Eprosartan mesylate 600 mg tablets were produced according to the principle of NAMADG in line with Example 2, but using the amounts of ingredients listed in the following Tables 2 and 3. According to these Examples 3 to 7, preferred amounts of liquid nonaqueous excipients selected from polysorbate, liquid polyethylene glycol, propylene glycol, glycerol and liquid paraffin were used, respectively, as a result of the test series described in Example 2.

**Table 2. Composition of Eprosartan 600 mg tablets with preferred amount of nonaqueos liquid excipient (Tween, PEG, Propylen glycol)**

| Constituent **Internals** | **Examples** | | | Function |
|---|---|---|---|---|
| | **3** | **4** | **5** | |
| Eprosartan mesylate | 735.80 mg | 735.80 mg | 735.80 mg | Active |
| Nonaqueos liquid excipient | Tween 80 55.00 mg | PEG-400 50.00 mg | Propylen glycol 60.00 mg | Binder, Wetting agent, Granulation agent |
| Macrogol 4000 (PEG-4000) | 36.00 mg | 15.00 mg | 35.00 mg | Binder |
| Isomalt (galenIQ721) | 46.70 mg | 36.70 mg | 48.70 mg | Filler |
| Crospovidone (Polyplasdone XL) | 40.00 mg | 50.00 mg | 40.00 mg | Disintegrant |
| Magnesium stearate | 15.00 mg | 15.00 mg | 15.00 mg | Lubricant |
| Talc | 15.00 mg | 15.00 mg | 15.00 mg | Glidant |
| Colloidal silicon dioxide (Aerosil 200) | 15.00 mg | 15.00 mg | 15.00 mg | Glidant |
| Sodium stearyl fumarate (Pruv) | 15.00 mg | 15.00 mg | 15.00 mg | Lubricant |

| Constituent **Externals** | | | | |
|---|---|---|---|---|
| Crospovidone (Polyplasdone XL) | 5.00 mg | 30.00 mg | 30.00 mg | Disintegrant |
| Macrogol 4000 (PEG-4000) | 8.00 mg | 5.00 mg | / | Binder |
| Isomalt (galenIQ721) | 10.00 mg | 10.00 mg | 10.00 mg | Filler |
| Colloidal silicon dioxide (Aerosil 200) | 1.00 mg | 2.50 mg | 2.50 mg | Glidant |
| Magnesium stearate | 2.50 mg | 5.00 mg | 5.00 mg | Lubricant |

**Table 3. Composition of Eprosartan 600 mg tablets with preferred amount of nonaqueos liquid excipient (Glycerol, Liquid paraffin)**

| Constituent **Internals** | **Examples** | | Function |
|---|---|---|---|
| | **6** | **7** | |
| Eprosartan mesylate | 735.80 mg | 735.80 mg | Active |
| Nonaqueos liquid excipient | Glycerol 70.00 mg | Liquid paraffin 45.00 mg | Binder, Wetting agent, Granulation agent |
| Macrogol 4000 (PEG-4000) | 20.00 mg | 20.00 mg | Binder |
| Isomalt (galenIQ721) | 40.70 mg | 64.20 mg | Filler |
| Crospovidone (Polyplasdone XL) | 30.00 mg | 40.00 mg | Disintegrant |
| Magnesium stearate | 15.00 mg | 15.00 mg | Lubricant |
| Talc | 15.00 mg | 15.00 mg | Glidant |
| Colloidal silicon dioxide (Aerosil 200) | 15.00 mg | 15.00 mg | Glidant |
| Sodium stearyl fumarate (Pruv) | 15.00 mg | 15.00 mg | Lubricant |

| Constituent **Externals** | | | |
|---|---|---|---|
| Crospovidone (Polyplasdone XL) | 20.00 mg | 10.00 mg | Disintegrant |
| Macrogol 4000 (PEG-4000) | 10.00 mg | 10.00 mg | Binder |
| Isomalt (galenIQ721) | 10.00 mg | 10.00 mg | Filler |
| Colloidal silicon dioxide (Aerosil 200) | 1.00 mg | 2.50 mg | Glidant |
| Magnesium stearate | 2.50 mg | 2.50 mg | Lubricant |

### Results of Examples 1 to 7:

Physical properties of briquetting powder as well as final mixture are important for a successful technological procedure using NAMADG. Therefore, some of the most important parameters have been observed and described below as well as presented in Figures and Table below.

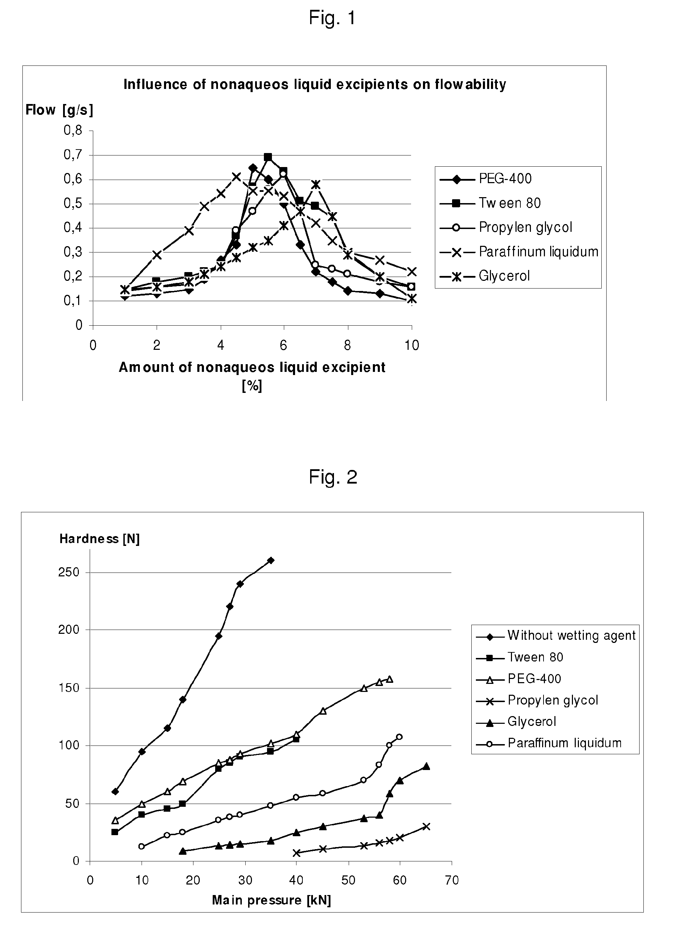

In particular, Figure 1 shows the basic effects of various nonaqueos liquid excipients and their contents on the flowability of powder mixture for briquetting. Fig. 2 shows the effects of various non-aqueous liquid excipients and their contents, as obtained from Examples 3 to 7 (cf. Tables 2 and 3), on the compressibility of powder mixtures for tabletting.

Further, the impact of various nonaqueos liquid excipients on the formulation of Eprosartan tablets obtained from Examples 3 to 7 (cf. Tables 2 and 3), referring to a matrix of 1000 mg (mass after NAMADG + briquetting + sieving + blending), are summarized in Table 4.

**Table 4**

| **Laboratory scale*** | Without Nonaqueos Liquid exc. | Tween 80 5.50% | PEG-400 5.00% | Propylen Glycol 6.00% | Glycerol 7.00% | Liquid paraffin 4.50% |
|---|---|---|---|---|---|---|
| Granulation moisture (%) | 0.95 | 0.97 | 1.36 | 5.00 | 4.25 | 1.97 |
| Flow density (ml/g) | 2.04 | 1.70 | 1.79 | 1.90 | 1.82 | 1.88 |
| Tapped density (ml/g) | 1.59 | 1.48 | 1.47 | 1.61 | 1.59 | 1.63 |
| Compressibility (%) | 22.10 | 16.95 | 17.60 | 15.30 | 16.90 | 19.80 |
| Flowability (g/sec) | 0.66 | 2.53 | 1.50 | 2.65 | 2.27 | 2.01 |
| Angle of repose (°) | 39.3 | 39.9 | 40.6 | 36.7 | 39.8 | 40.1 |
| **COMPRESSION** | | | | | | |
| Punches (mm x mm) | 19.8x8.5 | 19.8 x 8.5 | 19.8 x 8.5 | 19.8 x 8.5 | 19.8 x 8.5 | 19.8x8.5 |
| Main pressure, kN | 18-27 | 25-29 | 40-45 | 60-65 | 56-60 | 53-58 |
| Hardness, N | 140-220 | 80-90 | 110-130 | 20-30 | 40-70 | 70-100 |
| Friability % | 0.10 | 0.05 | 0.05 | > 1.00 | 0.55 | 0.40 |
| Disintegration time (min:s) | 3:30 | 8:00 | 6:00 | 0:30 | 1:30 | > 15 min |
| **Dissolution rate** | | | | | | |
| (0.1 M HCl) | % | % | % | % | % | % |
| 10 minutes | 35.1 | 40.0 | 22.6 | 49.9 | 46.6 | 5.0 |
| 15 minutes | 41.3 | 48.2 | 34.0 | 57.0 | 55.0 | 9.3 |
| 30 minutes | 53.7 | 58.1 | 53.7 | 70.2 | 68.1 | 13.4 |
| 45 minutes | 61.3 | 63.2 | 64.3 | 76.0 | 74.2 | 17.8 |
| 60 minutes | 66.4 | 68.0 | 71.1 | 79.3 | 78.3 | 21.3 |
| 90 minutes | 72.9 | 70.9 | 79.3 | 83.2 | 82.1 | 30.9 |

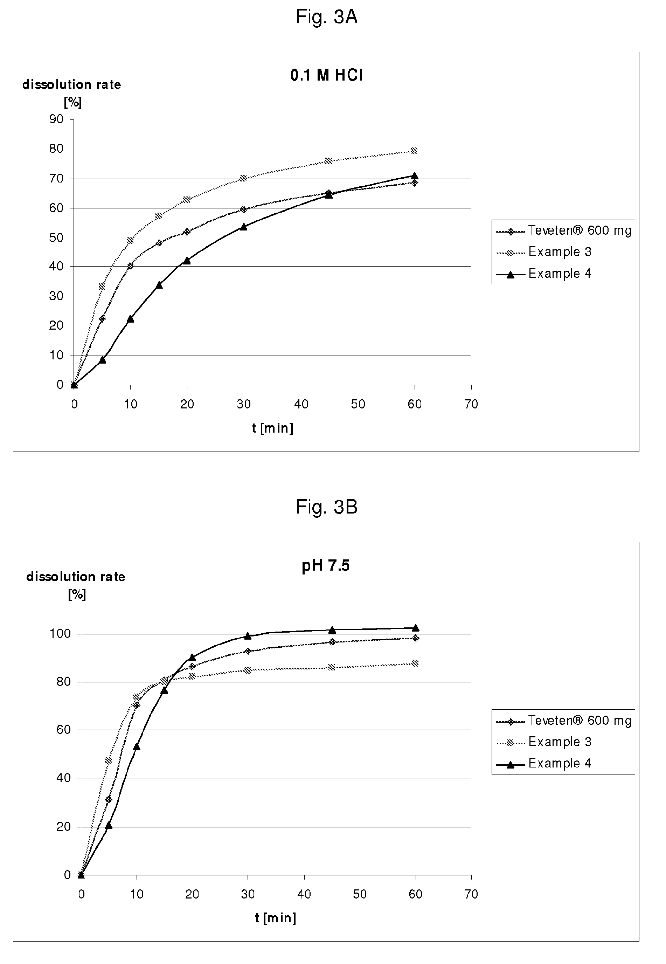

The dissolution properties of eprosartan mesylate from the tablets produced in Examples 3 and 4 were evaluated by *in vivo* relevant dissolution test. USP apparatus 2 was used for testing. 1000 mL of artificial juice (0.1 M HCl; or potassium phosphate buffer pH 7.5) is placed in the dissolution vessel, mixed with a paddle at 50 rpm and kept at 37 ± 0.5°C. Samples were taken from the dissolution vessel at regular time intervals and the concentrations of eprosartan mesylate were analyzed by HPLC.
The results are shown in Figure 3A (at 0.1 M HCl) and Figure 3B (at pH 7.5) when using reference product (Teveten) and, as samples of the present invention, either polysorbate/Tween 80 or PEG-400 as liquid nonaqueous excipient.

Dissolution profiles for the samples of Examples 3 and 4 have shown that the liquid nonaqueous excipient (here: Tween 80 and macrogol 400 (PEG-400) have very significant influence on dissolution profiles (*in vitro* studies). On the other hand, without these excipients the physical properties of powder mixture for briquetting and granulate for tableting were significantly inferior, which appeared to be mainly due to very low compressibility and consequently low weight and high variability or lack of reproducibility of tablets.

According to very low flowability of eprosartan mesylate (below 0.15 g/sec; with sticking on punches) and high percentage of active principle (≥ 73%) in comparison to excipients, direct compression with most commonly used binders in different combinations was not possible without nonaqueos liquid excipient. On the other hand, the use of nonaqueos liquid excipient significantly contributed to improved flowabiltiy and compressibility of the API, especially when added in small amount of up to 10 wt.-%.

The use of macrogol-4000 or isomalt with nonaqueos liquid excipient excipients, especially when used in combination, significantly further improved flowability and compressibility (*p < 0.05*; Student's ttest), compared to mixtures for directly compression.

Physical properties of powders and granulates over and above dissolution profiles for the mixtures of Examples have shown that nonaqueos liquid excipients have very significant influence (*p < 0.05* ; Student's *t* test) on briquetting and tableting processes (in terms of technical performances) as well as *in vitro* studies (in terms of dissolution profiles).

The compressibility was used as a measure of the flow properties of the granulations. The lower compressibility the better flow properties. It was found by one-way ANOVA that the compressibility was affected by all the variables: formulation (*p < 0.001*), nonaqueos liquid excipient content (*p < 0.001*) and wet massing time (*p < 0.05*). Wet massing time means the time of mixing after the liquid excipient has been added. The preferred wet massing time is 2 to 5 minutes, optimal time is around 3 minutes (e.g. 2.5 to 3.5 minutes).

The mass variations were found to vary between 0.5 and 5.1% (RSD), and the best results were with PEG-400, Tween 80, propylen glycol, glycerol, liquid paraffin in amount of 5.0, 5.5, 6.0, 7.0, and 4.5%, respectively. It was impossible to make tablets containing more than 70% of voluminous API with acceptable flowability and compressibility, with a crushing strength of more than about 50 N. Using combination of nonaqueos liquid excipient/PEG-4000 in the ratio from 1/10 to 10/1 gives excellent properties of final tablet mass (≥ 1.60 g/s; 15-20%; 30-40°) with crushing strength of tablets between 75 and 200 N.

The friability was found to be affected by the crushing strength of the tablets (*p < 0.001*) and the formulation (*p < 0.001*). A higher crushing strength and an increasing content of PEG-4000 and isomalt reduced the friability. At the high level of crushing strength, the friability was lower than 0.1% with optimal PEG-4000 and isomalt content in formulation.

In addition, the following general observations can be inferred from our results:

An API formulation can be provided in the form of a free-flowing, cohesive briquetting powder and nonaqueos dry granulate, capable of being further processed by dry granulation or directly compressed into a tablet.

The NAMADG procedure according to the present invention can be used alone or in combination with briquetting.

An API formulation can be provided in unit dosage form having acceptable dissolution profiles, acceptable degrees of hardness (50-200 N) and resistance to chipping, as well as short disintegration time (< 15 min).

Eprosartan mesylate can be processed in preferred anhydrous form, and the process involving NAMADG leads to useful particle size lower than 20 µm

Nonaqueos liquid excipients, especially in appropriate amounts (1-10%) is significant to produce powder with acceptable flowability (≥ 0.5 g/s), compressibility (final granulate; 15-20%) and tablet characteristics (hardness, friability, weight, shape, disintegration, dissolution).

Best results are achieved when using appropriate amount of nonaqueos liquid excipients (1-10 %) selected from polysorbate (Tween^{™} 20, 60, 65, 80, 85), glycerol, liquid polyethylene glycol (macrogol; PEG-200, PEG-400, PEG-600), liquid paraffin, and propylene glycol.

The NAMADG procedure according to the present invention is highly advantageous for high API dosage forms, for example with more than 60 wt.%, more preferably more than 2/3 of the total amount, and even more than 70 wt.% API in the formulation.

The NAMADG procedure according to the present invention is therefore also very useful for powders of API having very poor flowability and inherently insufficient compression properties.

Considering the last two issues, the NAMADG procedure according to the present invention are considered particularly suitable for producing tablet formulations comprising eprosartan, irbesartan, eprosartan/hydrochlorothiazide, irbesartan/hydrochlorothiazide, Co-amoxiclav, levofloxacin, metformin, rosiglitazon/metformin or sevelamer.

### Example 8:

Tablets of eprosartan as API, admixed with Tween as nonaqueous liquid excipient, were obtained by a process according to the principle of NAMADG in line with Example 2 and using the ingredients and tablet composition shown in Table 5 below.

**Table 5**

| **Composition Internals** | **Content [%]** | **Function** |
|---|---|---|
| API (Eprosartan - d(0.9) = 10 µm) | 73.58 | Active |
| Crospovidone (Polyplasdone XL) | 4.00 | Disintegrant |
| Nonaqueos liquid excipient* | Up to 10.00 | Binder, Wetting agent, Granulation agent |
| Macrogol 4000 (PEG-4000) | 3.6 | Binder |
| Isomalt (galenIQ721) | Up to 10.00 | Filler |
| Magnesium stearate | 1.50 | Lubricant |
| Talc | 1.50 | Glidant |
| Colloidal silicon dioxide (Aerosil 200) | 1.50 | Glidant |
| Sodium stearyl fumarate (Pruv) | 1.50 | Lubricant |

| Constituent **Externals** | | |
|---|---|---|
| Magnesium stearate | 0.25 | Lubricant |
| Crospovidone (Polyplasdone XL) | 0.50 | Disintegrant |
| Macrogol 4000 (PEG-4000) | 0.80 | Binder |
| Isomalt (galenIQ721) | 1.00 | Filler |
| Colloidal silicon dioxide (Aerosil 200) | 0.10 | Glidant |
| | 100.00 | |

| | | |
|---|---|---|
| ** polysorbate (Tween^{™} 20, 60, 65, 80, 85)* | | |

### Example 9:

Tablets of eprosartan as API, admixed with Macrogol as nonaqueous liquid excipient, were obtained by a process according to the principle of NAMADG in line with Example 2 and using the ingredients and tablet composition shown in Table 6 below.

**Table 6**

| **Composition Internals** | **Content [%]** | **Function** |
|---|---|---|
| API (Eprosartan - d(0.9) = 10 µm) | 73.58 | Active |
| Crospovidone (Polyplasdone XL) | 5.00 | Disintegrant |
| Nonaqueos liquid excipient* | Up to 10.00 | Binder, Wetting agent, Granulation agent |
| Macrogol 4000 (PEG-4000) | 1.50 | Binder |
| Isomalt (galenIQ721) | Up to 10.00 | Filler |
| Magnesium stearate | 1.50 | Lubricant |
| Talc | 1.50 | Glidant |
| Colloidal silicon dioxide (Aerosil 200) | 1.50 | Glidant |
| Sodium stearyl fumarate (Pruv) | 1.50 | Lubricant |

| Constituent **Externals** | | |
|---|---|---|
| Magnesium stearate | 0.50 | Lubricant |
| Crospovidone (Polyplasdone XL) | 3.00 | Disintegrant |
| Macrogol 4000 (PEG-4000) | 0.50 | Binder |
| Isomalt (galenIQ721) | 1.0 | Filler |
| Colloidal silicon dioxide (Aerosil 200) | 0.25 | Glidant |
| | 100.00 | |

| | | |
|---|---|---|
| ** macrogol (PEG-200, PEG-400, PEG-600)* | | |

### Example 10:

Tablets of eprosartan as API, admixed with glycerol as nonaqueous liquid excipient, were obtained by a process according to the principle of NAMADG in line with Example 2 and using the ingredients and tablet composition shown in Table 7 below.

**Table 7**

| **Composition Internals** | **Content [%]** | **Function** |
|---|---|---|
| API (Eprosartan - d(0.9) = 10 µm) | 73.58 | Active |
| Crospovidone (Polyplasdone XL) | 3.00 | Disintegrant |
| Nonaqueos liquid excipient* | Up to 10.00 | Binder, Wetting agent, Granulation agent |
| Macrogol 4000 (PEG-4000) | 2.00 | Binder |
| Isomalt (galenIQ721) | Up to 10.00 | Filler |
| Magnesium stearate | 1.50 | Lubricant |
| Talc | 1.50 | Glidant |
| Colloidal silicon dioxide (Aerosil 200) | 1.50 | Glidant |
| Sodium stearyl fumarate (Pruv) | 1.50 | Lubricant |

| Constituent **Externals** | | |
|---|---|---|
| Magnesium stearate | 0.25 | Lubricant |
| Crospovidone (Polyplasdone XL) | 2.00 | Disintegrant |
| Macrogol 4000 (PEG-4000) | 1.00 | Binder |
| Isomalt (galenIQ721) | 1.00 | Filler |
| Colloidal silicon dioxide (Aerosil 200) | 0.10 | Glidant |
| | 100.00 | |

| | | |
|---|---|---|
| ** glycerol* | | |

### Example 11:

Tablets of eprosartan as API, admixed with propylene glycol as nonaqueous liquid excipient, were obtained by a process according to the principle of NAMADG in line with Example 2 and using the ingredients and tablet composition shown in Table 8 below.

**Table 8**

| **Composition Internals** | **Content [%]** | **Function** |
|---|---|---|
| API (Eprosartan - d(0.9) = 10 µm) | 73.58 | Active |
| Crospovidone (Polyplasdone XL) | 4.00 | Disintegrant |
| Nonaqueos liquid excipient* | Up to 10.00 | Binder, Wetting agent, Granulation agent |
| Macrogol 4000 (PEG-4000) | 3.50 | Binder |
| Isomalt (galenIQ721) | Up to 10.00 | Filler |
| Magnesium stearate | 1.50 | Lubricant |
| Talc | 1.50 | Glidant |
| Colloidal silicon dioxide (Aerosil 200) | 1.50 | Glidant |
| Sodium stearyl fumarate (Pruv) | 1.50 | Lubricant |

| Constituent **Externals** | | |
|---|---|---|
| Magnesium stearate | 0.50 | Lubricant |
| Crospovidone (Polyplasdone XL) | 3.00 | Disintegrant |
| Macrogol 4000 (PEG-4000) | / | Binder |
| Isomalt (galenIQ721) | 1.00 | Filler |
| Colloidal silicon dioxide (Aerosil 200) | 0.25 | Glidant |
| | 100.00 | |

| | | |
|---|---|---|
| ** propylene glycol* | | |

### Example 12:

Tablets of eprosartan as API, admixed with liquid paraffin as nonaqueous liquid excipient, were obtained by a process according to the principle of NAMADG in line with Example 2 and using the ingredients and tablet composition shown in Table 9 below.

**Table 9.**

| **Composition Internals** | **Content [%]** | **Function** |
|---|---|---|
| API (Eprosartan - d(0.9) = 10 µm) | 73.58 | Active |
| Crospovidone (Polyplasdone XL) | 4.00 | Disintegrant |
| Nonaqueos liquid excipient* | Up to 10.00 | Binder, Wetting agent, Granulation agent |
| Macrogol 4000 (PEG-4000) | 2.00 | Binder |
| Isomalt (galenIQ721) | Up to 10.00 | Filler |
| Magnesium stearate | 1.50 | Lubricant |
| Talc | 1.50 | Glidant |
| Colloidal silicon dioxide (Aerosil 200) | 1.50 | Glidant |
| Sodium stearyl fumarate (Pruv) | 1.50 | Lubricant |

| Constituent **Externals** | | |
|---|---|---|
| Magnesium stearate | 0.25 | Lubricant |
| Crospovidone (Polyplasdone XL) | 1.00 | Disintegrant |
| Macrogol 4000 (PEG-4000) | 1.00 | Binder |
| Isomalt (galenIQ721) | 1.00 | Filler |
| Colloidal silicon dioxide (Aerosil 200) | 0.25 | Glidant |
| | 100.00 | |

| | | |
|---|---|---|
| ** liquid paraffin* | | |

### Example 13:

In order to test the ability of the NAMADG process principle according to the present invention for preparing tablets without occurrence different polymorphic forms of the API in the product, a placebo powder without API and anhydrous eprosartan mesylate processed according to Examples 2 to 7 were investigated. All examples were checked for eprosartan form. For comparison tests were conducted after conventional wet granulation conditions.

X-ray diffraction patterns of the placebo powder as well as of tablets exhibited characteristic diffraction lines corresponding to anhydrous eprosartan mesylate. As a result, physical stability of the eprosartan mesylate was proven during processes when using the most critical API form, i.e. the anhydrate. Anhydrous Eprosartan was found to be physically and chemically stable at ambient temperature and humidity used throughout the NAMADG-based method. The final tablet form and placebo powder were analyzed by X-ray diffraction (XRD) pattern to confirm presence of anhydrous form of eprosartan mesylate. More generally, X-ray diffraction (XRD) pattern can be obtained by methods known in the art, for example using Philips X'Pert PRO diffractometer with X'Celerator detector while exposing the searched material to CuKa radiation.

In the comparison case the wet granulation lead to the formation to all three investigated forms, namely anhydrous-, mono- and dihydrate. As monohydrate is not stable at room conditions, it was further transformed to anhydrous or dehydrate, meaning that the equilibrium reaction is constantly going on. In solid dosage form which was prepared by wet granulation, proportional amounts of three different hydrated forms occurred dependent on many factors such as amount of moisture, working environment, drying time of granulate and so on. That kind of variation might affect *in vitro* and *in vivo* properties, and may have negative influence during a tableting process due to variations of weight and hardness of the tablets. In our case anhydrous form of eprosartan mesylate was confirmed in tablets when using Tween, PEG, and liquid paraffin as selected nonaqueous liquid excipient. On the other hand, according to high higroscopicity and water activity of more than 0.7 of propylen glycol and glycerol, substantial amounts of hydrated forms of API were found in their formulations. Thus, water activity is a critical factor to be observed for the nonaqueous liquid excipient and other optional excipients in cases of selecting hygroscopic APIs like eprosartan. It is considered that a maximum limit of water activity of each excipient to be less than 0.62, preferably less than 0.60 and more preferably less than 0.50, respectively determined at room temperature, is significant to keep the initial polymorphic form of API without substantial variation/interchanging during the granulation process.

To conclude: dry granulation (no water added) one ends up with only anhydrous eprosartan. On the other hand, wet granulation (combined with heavy drying) leads to anhydrous form partly, but there are substantial amounts of dihydrate and minute amounts of monohydrate present as well.

Thus, the invention is capable of providing a process for preparing a compressed API tablet by NAMADG in unit dosage form, without physical change of active principle form, specifically demonstrated by maintaining anhydrous against hydrated forms (especially mono- and di-hydrated forms).

## Claims

1. A dry formulation or granulation comprising, in admixture, more than 50 wt.% of active pharmaceutical ingredient and from 1 to 10 wt.% of nonaqueous excipient selected from liquid substances.

2. A dry formulation or granulation comprising, in admixture and without water or lower alcohol having been added in any step of its preparation, (i) more than 50 wt.% of active pharmaceutical ingredient and (ii) a nonaqueous excipient selected from liquid substances; wherein the liquid nonaqueous excipient is adsorbed to dryness by the active pharmaceutical ingredient and/or by a further solid excipient.

3. The dry formulation or granulation according to claim 1 or 2, wherein the nonaqueous excipient is selected from liquid surfactants, preferably of non-ionic type; liquid polyethylene glycol; liquid paraffin; propylene glycol; liquid fatty alcohols with at least 8 carbon atoms; liquid triglycerides or oils; liquid wax; liquid polyethoxylated fatty acids; liquid PEG glycerol fatty acid esters; and liquid ethers of polyethylene glycol and alkyl alcohols; preferably selected from the group consisting of polysorbate, glycerol, liquid polyethylene glycol, liquid paraffin and propylene glycol.

4. The dry formulation or granulation according to any one of claims 1 to 3, further comprising a solid excipient adsorbing the originally liquid nonaqueous excipient.

5. The dry formulation or granulation according to any one of the preceding claims, wherein no water, aqueous solution, lower alcohol or lower alcoholic solution has been added to the dry formulation during its preparation.

6. The dry formulation or granulation according to any one of the preceding claims, further comprising a solid excipient selected from microcrystalline cellulose, lactose, isomalt, solid polyethylene glycol, mannitol, sorbitol, starch, calcium phosphates, carboxymethylcellulose (calcium or sodium), cellulose, silicified microcrystalline cellulose, cellulose acetate, colloidal silicon dioxide, dextranes, dextrin, glucose, ethylcellulose, fructose, hydroxyethyl cellulose, hydroxyethylmethyl cellulose, hydroxypropyl cellulose, hypromellose, lactitol, magnesium carbonate, maltodextrin, maltose, methylcellulose, polydextrose, pregelatinized starch, zein, and calcium silicate, preferably selected from solid polyethylene glycol, isomalt, microcristalline cellulose, mannitol and lactose.

7. The dry formulation or granulation according to any one of the preceding claims, wherein nonaqueous, liquid excipient is mixed with solid excipient at a weight ratio of 1:10 to 10:1.

8. The dry formulation or granulation according to any one of the preceding claims, wherein active pharmaceutical ingredient is selected from a group of eprosartan, irbesartan, amoxiciline, levofloxacine, metformin and sevelamer, their salts, hydrates or solvates, optionally combined with another active pharmaceutical ingredient.

9. A process for the preparation of a pharmaceutical formulation, comprising mixing more than 50 wt.% of active pharmaceutical ingredient with from 1 to 10 wt.% of a nonaqueous liquid excipient to obtain a dry or moisture-activated formulation, and subjecting the thus obtained formulation to processing to prepare the pharmaceutical formulation.

10. A process for the preparation of a pharmaceutical formulation, comprising subjecting an active pharmaceutical ingredient to granulation with a nonaqueous liquid excipient, without adding anyone of water, an aqueous solution, lower alcohol or lower alcoholic solution, as granulation liquid, wherein the nonaqueous liquid excipient and other optional excipients each has a water activity of less than 0.62 determined at room temperature.

11. The process according to claim 9 or 10, wherein the nonaqueous excipient is selected from surfactants, preferably of non-ionic type; liquid polyethylene glycol; liquid paraffin; propylene glycol; liquid fatty alcohols with at least 8 carbon atoms; liquid triglycerides or oils; liquid wax; liquid polyethoxylated fatty acids; liquid PEG glycerol fatty acid esters; and ethers of polyethylene glycol and alkyl alcohols; preferably selected from, preferably selected from the group consisting of polysorbate, glycerol, liquid polyethylene glycol, liquid paraffin and propylene glycol, more preferably selected from polysorbate and liquid polyethylene glycol alone or together with other pharmaceutically acceptable excipients.

12. The process according to any one of claims 9 to 11, further comprising admixing with a solid excipient capable adsorbing the liquid nonaqueous excipient, and optionally admixing with other solid pharmaceutically acceptable excipients;
wherein the solid excipient capable adsorbing the liquid nonaqueous excipient is preferably selected from the group consisting of microcrystalline cellulose, lactose, isomalt, solid polyethylene glycol, mannitol, sorbitol, starch, calcium phosphates, carboxymethylcellulose (calcium or sodium), cellulose, silicified microcrystalline cellulose, cellulose acetate, colloidal silicon dioxide, dextranes, dextrin, glucose, ethylcellulose, fructose, hydroxyethyl cellulose, hydroxyethylmethyl cellulose, hydroxypropyl cellulose, hypromellose, lactitol, magnesium carbonate, maltodextrin, maltose, methylcellulose, polydextrose, pregelatinized starch, zein, and calcium silicate; more preferably selected from solid polyethylene glycol, isomalt, microcristalline cellulose, mannitol and lactose.

13. The process according to any one of claims 9 to 12, wherein the active pharmaceutical ingredient has a property of existing in at least two polymorphic forms, while the form subjected to granulation is provided in a single first form, whereupon an interconversion from said first form to another or mixed form of the active pharmaceutical ingredient is prevented during granulation, preferably also during the optionally steps of briquetting or slugging, sieving or milling, and tabletting.

14. The process according to any one of claims 9 to 13, further the processing to prepare the pharmaceutical formulation involves direct compression or dry granulation process, optionally further comprising tabletting.

15. The process according to any one of claims 9 to 14, wherein active pharmaceutical ingredient is selected from a group of eprosartan, irbesartan, amoxiciline, levofloxacine, metformin and sevelamer, their salts, hydrates or solvates, optionally combined with another active pharmaceutical ingredient.

16. A pharmaceutical formulation, comprising a dry formulation or granulation according to any one of claims 1 to 8, or obtained by a process according to any one of claims 8 to 15.

17. The pharmaceutical formulation according to claim 16, which is a tablet, wherein the table preferably has a hardness of between 40 and 180 N.

18. Dry formulation or granulation according to any of claims 1 to 8, or pharmaceutical formulation according to claim 16 or 17 for use in the treatment of human or animal disease.
